(19) Europäisches Patentamt

European Patent Office

Office européen des brevets

(11) Publication number: **0 382 578 B1**

## (12) EUROPEAN PATENT SPECIFICATION

(45) Date of publication of patent specification: **13.09.95**

(21) Application number: **90301463.7**

(22) Date of filing: **12.02.90**

(51) Int. Cl.⁶: **A23L 1/308**, A23L 1/0534, A21D 13/08, A23L 1/24, A23D 7/00

(54) **Substitution of degraded cellulose derivatives for high calorie ingredients in foods.**

(30) Priority: **10.02.89 US 309387**
**23.06.89 US 370629**
**12.01.90 US 464291**

(43) Date of publication of application:
**16.08.90 Bulletin 90/33**

(45) Publication of the grant of the patent:
**13.09.95 Bulletin 95/37**

(84) Designated Contracting States:
**AT BE CH DE DK ES FR GB GR IT LI LU NL SE**

(56) References cited:
**EP-A- 0 251 798          DE-A- 2 016 203**
**DE-C- 667 864            FR-A- 1 363 884**
**GB-A- 1 122 006          GB-A- 1 423 608**

**CHEMICAL ABSTRACTS, vol. 101, 1984, page 81, abstract no. 56734p, Columbus,Ohio, US; & JP-A 59 41 301**

(73) Proprietor: **Alko Ltd.**
**P.O. Box 350**
**SF-00101 Helsinki (FI)**

(72) Inventor: **Bagley, Lindsey**
**4 Barn Close**
**Maidenhead**
**Berks. SL6 7HD (GB)**
Inventor: **Lindley, Michael**
**17 Highway,**
**Edgecomb Park**
**Crowthorne, Berks. RG11 6HE (GB)**
Inventor: **Bosdet, Sarah**
**45 All Saints Close, Glebe Park**
**Wokingham, Berks. RG11 1WE (GB)**
Inventor: **Timonen, Maritta**
**c/o Alko Ltd.,**
**P.O.B. 350**
**SF-00101 Helsinki (FI)**
Inventor: **Turunen, Marja**
**c/o Alko Ltd.,**
**P.O.B. 350**
**SF-00101 Helsinki (FI)**
Inventor: **Vaara, Timo**
**c/o Alko Ltd.,**
**P.O.B. 350**
**SF-00101 Helsinki (FI)**

Inventor: **Vaara, Martti**
**c/o Alco Ltd.,**
**P.O.B. 350**
**SF-00101 Helsinki (FI)**
Inventor: **Lahtinen, Tarja**
**c/o Alko Ltd.,**
**P.O.B. 350**
**SF-00101 Helsinki (FI)**


(74) Representative: **Collier, Jeremy Austin Grey et al**
**J.A.KEMP & CO.**
**14, South Square**
**Gray's Inn**
**London WC1R 5LX (GB)**

**Description**

This invention relates to novel degradation products of cellulose derivatives, the substitution thereof in food products for a substantial portion of the normal fat, sugar, carbohydrate or other high caloric content of a food product, and methods for producing such degradation products and low calorie food products therefrom.

Cellulose derivatives such as carboxymethylcellulose, methylcellulose, methylethylcellulose, hydroxypropylmethylcellulose and hydroxypropylcellulose are non-caloric (non-metabolizable by humans or intestinal flora in human beings), odorless, tasteless water-soluble polymers derived from cellulose. These cellulose derivatives may act as thickeners, binders, stabilizers, suspending agents or flow control agents. They form films resistant to oils, greases and organic solvents.

They dissolve rapidly in cold and hot water and are physiologically inert. In theory, the non-caloric nature of cellulose derivatives would suggest that they might be used as filler materials or substitutes for fat, sugar, carbohydrate or other high calorie components of normal food products. However, the simple substitution of such non-toxic non-caloric substances for a high calorie food component, is not practicable because any substantial substitution of a normal food ingredient will typically alter one or more of the color, volume, texture, structure, mouthfeel, odor or flavor of the food to such an extent as to render the food product unacceptable to a consumer.

Degradation of cellulose derivatives is normally considered undesirable and to be avoided. Cellulolytic and viscosity reducing treatments on cellulose derivatives have been deliberately avoided in the past and high molecular weight products deliberately produced. Indeed, non-degraded cellulose derivatives have been incorporated into food stuff compositions as disclosed in U.S Patent No. 4,214,009 to Chang.

Enzymatic hydrolysis of cellulose derivatives have been studied in the past in the context of synergism studies among combinations of enzymes, the possible indexing of substituent distribution patterns, the effect of various substituents on enzymatic hydrolysis and the like. Such studies have been published in the following: Chouchon et al., Biotech. Bioeng., Vol. 26, pp. 988-991 (1984); Henrissat et al., Biotechnology, Vol. 3, pp. 722-726 (1985); Chetkarov et al., Monatshefte Fur Chemie, Vol. 116, pp. 1433-45 (1985); Chetkarov et al., Monatshefte Fur Chemie, Vol. 117, pp. 1021-1026 (1986); Wirick, J. Polym. Sci., Part A-1, Vol. 6, pp. 1195-1974 (1968); Bhattacharjee, J. Polym. Sci., Part C, Vol. 36, pp. 509-521 (1971). Reduction of chain length determinations have also been studied. Almin et al., Arch. Biochem. Biophys., pp. 124, 129 (1968); Ghose, Biotech. Bioeng., Vol. 11, pp. 239 (1969).

The present invention also deals with a novel degradation of cellulose derivatives to produce novel low molecular weight polymers or oligomeric mixtures which can be used to replace a substantial portion of high caloric ingredients in conventional food recipes and obtain an end food product which is acceptable to the consumer in terms of eating quality, i.e. flavor, odor, mouthfeel, texture, etc. These relatively low molecular weight polymer, oligomeric mixtures and fractions (of the total mixture of oligomers into further separated mixtures of oligomers of varying chain length) thereof are more advantageous for applications in food than the high molecular weight cellulose derivative.

## SUMMARY OF THE INVENTION

The invention discloses the use and manufacture of novel water soluble mixtures of relatively low molecular weight polymers or oligomers derived from cellulose derivatives as well as fractions of the mixtures of oligomers obtained from the initial degradative process.

The oligomeric mixtures can be made from several different cellulose derivatives, the most preferred raw material being carboxymethylcellulose. The oligomeric mixtures can be prepared by different modified and unmodified cellulolytic enzymes, the most preferred sources of the enzyme being strains of Trichoderma reesei, Aspergillus and Penicillium.

The oligomeric mixtures and fractions thereof have a variety of applications, but they are especially useful as fat, carbohydrate, high calorie ingredient, calorie saving or low caloric substitutes in a wide range of food stuffs. For this purpose preferably less than about 75% of the high calorie ingredient, but usually at least about 25%, is removed from the food composition.

The present invention makes use of a water-soluble mixture of oligomers derived from a water-soluble mixture of oligomers derived from a water-soluble cellulose derivative comprising a cellulose derivative enzymatically degraded to form a mixture of oligomers having an average degree of polymerization in the range of 3 to 100, preferably 5 to 100, and an average molecular weight of 500 to 33,000. The water-soluble cellulose derivative is selected from the group of carboxylmethylcellulose, methylcellulose, methylethylcellulose, hydroxypropylmethylcellulose and hydroxypropylcellulose and mixtures thereof.

The cellulose derivative is degraded by an enzyme preparation, typically selected from the group of cellulases, modified cellulases and mixtures thereof.

An enzyme preparation may be a cellulase or modified cellulase (i.e., modified to remove or prevent the formation of mono- and disaccharides producing enzymes in the cellulase preparation in the first instance, e.g., by genetic alteration of the microorganism from which the cellulase preparation is prepared) preferably produced from microorganisms selected from the group of Trichoderma, Aspergillus and Penicillium. Most preferably a cellulase preparation is derived from Trichoderma reesei from which at least one of beta-glucosidase and cellobiohydrolase activities have been removed. An enzyme preparation most preferably comprises endo-1,4-beta-glucanase.

The water-soluble oligomers of the aforesaid cellulose derivatives are produced by a method comprising the steps of: selecting a water soluble cellulose derivative; selecting a cellulolytic enzyme which degrades the selected cellulose derivative into a mixture of oligomers having an average degree of polymerization in the range of 3 to 100 and a molecular weight in the range of 500 to 33,000; and reacting the selected cellulolytic enzyme with the selected cellulose derivative for a time and at a temperature sufficient to produce the mixture of oligomers. The cellulose derivative is selected from the group of carboxymethylcellulose, methylcellulose, methylethylcellulose, hydroxypropylmethylcellulose, hydroxypropylcellulose and mixtures thereof.

The cellulolytic enzyme may be selected by selecting a microorganism which produces a cellulolytic enzyme and preparing a cellulolytic enzyme from a culture of the microorganism. The cellulolytic enzyme produced by the microorganism may be purified to remove enzymes which will react with the cellulose derivative to produce mono- and disaccharides. The microorganism is preferably selected from the group of Trichoderma, Aspergillus and Penicillium. In order to prevent hydrolysis of the cellulose derivative into mono- and disaccharides the selected microorganism may alternatively be treated to alter the genes of the microorganism such that production of mono- and disaccharide generating enzymes by the genes is disenabled.

The invention further contemplates removing all or a portion (typically up to 50% by weight) of a selected fat contained in a foodstuff and substituting a mixture of oligomers produced according to the invention for the removed fat; and/or, removing up to about 40% of a selected carbohydrate contained in a foodstuff and substituting a mixture of oligomers produced according to the invention for the removed carbohydrate.

The invention also contemplates separating a mixture of oligomers initially produced by a cellulolytic agent or process according to the invention into fractions of oligomers of different average molecular weight, removing all or at least a portion (typically up to 50% by weight) of a selected fat contained in a foodstuff, and substituting one or more of the fractions for the removed fat; and/or, separating a mixture of oligomers initially produced by a cellulolytic agent or process according to the invention into fractions of oligomers of different average molecular weight, removing up to 40% by weight of a selected carbohydrate contained in a foodstuff and substituting one or more of the fractions for the removed carbohydrate.

BRIEF DESCRIPTION OF THE DRAWINGS

FIG. 1 shows molecular weight distribution patterns of a methylcellulose and its hydrolysate as described in Example 2a herein;

FIG. 2 shows molecular weight distribution patterns of hydroxypropylmethylcellulose and its hydrolysate as described in Example 2b herein;

FIG. 3 shows molecular weight distribution patterns of a carboxymethylcellulose and its hydrolysate as described in Example 2c(i) herein;

FIG. 4 shows molecular weight distribution patterns of a carboxymethylcellulose and its hydrolysate as described in Example 3 herein;

FIG. 5 shows molecular weight distribution patterns of selected fractions of the carboxymethylcellulose hydrolysate as described in Example 3 herein.

DETAILED DESCRIPTION OF THE INVENTION

This invention relates to a water soluble or suspendable mixture of oligomers derived from a cellulose derivative and its fractions. The oligomeric mixtures are characterized by having an average degree of polymerization (DP) in the range of 3-100 and a molecular weight in the range of 500-33,000.

Following is a description of some exemplary embodiments of the invention where a cellulolytic treatment is carried out using enzymatic methods. The following description includes a most preferred protocol for initial preparation of an enzyme.

Enzyme Preparation

Enzymes which may be used in this invention are various food-grade cellulase preparations. They can be produced from a multitude of different microorganisms such as strains of Trichoderma, Aspergillus, Penicillium, etc. A selected microorganism strain is grown by conventional means in a medium containing food grade materials such that the cellulases are produced, the microorganism is separated from the medium, the medium is collected and typically concentrated and dried. These enzymes can be used as such or in mixtures and they can be modified in many different ways known to the man skilled in the art. A most preferred enzyme preparation is produced from Trichoderma reesei, from which preparations the beta-glucosidase and/or the cellobiohydrolase activities are removed chromatographically or genetically. Beta-glucosidase and/or cellobiohydrolase activities are preferably removed from the selected cellulase preparation so as to prevent the degradation of the cellulose derivative into mono- and disaccharides. Genetic alteration of the appropriate enzyme producing microorganism may be effected with radiation or mutagenic chemical agents (or by gene inactivation by recombinant DNA methods) so as to disenable production of beta-glucosidase and cellobiohydrolase by the microorganism. Cellulase preparations suitable for use herein are, e.g., the commercially available cellulase preparations designated as the Econase series as produced by Alko Ltd., Helsinki Finland.

Starting Materials

Cellulose derivatives for use herein are carboxymethyl-, methyl-, methylethyl-, hydroxypropylmethyl- or hydroxypropylcellulose and any combinations thereof.

General Preparation of a Typical Hydrolysate

In one embodiment of the invention, cellulose derivative hydrolysates may be prepared from soluble cellulose derivatives as defined above by an enzymatic hydrolysis utilizing a cellulase preparation having endo-1,4-beta-glucanase as the sole active hydrolytic agent such that only insignificant amounts of mono- and disaccharides which are absorbed in human intestine (e.g., glucose) or hydrolyzed by the intestinal bacterial flora (e.g., cellobiose), are produced. On the other hand the average degree of polymerization (DP) of the oligomers formed by such a hydrolysis is lower than 100, and thus the viscosity of solutions of the hydrolysate is reduced significantly compared to the viscosity of solutions of the unhydrolysed cellulose derivatives. The specific conditions suitable for and the specific time sufficient to secure the desired hydrolysis may be readily determined for each selected cellulose derivative and each selected enzyme preparation.

USE OF OLIGOMERIC MIXTURES DERIVED FROM CELLULOSE DERIVATIVES

The degraded cellulose derivative products used in the invention (and fractions thereof) dissolve rapidly in cold and hot water and are physiologically inert. Such initially formed oligomeric mixtures and selected fractions may act as thickeners, binders (e.g. in coating applications such as in the formation of conductive particle filled coatings on electrodes), stabilizers, suspending agents or flow control agents, or fillers in wide variety of applications such as in cosmetics, pharmaceuticals, plastics, paper and the like. Such products may also form films resistant to oils, greases and organic solvents and may be useful as organic resistant coatings such as coatings on clothing, paper and the like.

The cellulose derivatives used as starting materials in the present invention are as such non-caloric. Because a degradative treatment according to the present invention does not produce significant amounts of metabolizable sugars, the resulting oligomeric mixtures according to this invention with their improved properties, are especially useful as low-caloric substitutes in food stuffs.

The oligomeric mixtures and fractions thereof produced according to this invention can be used for example as new low-caloric fat sparing agents or bulking agents. These mixtures can be used to replace fat in various food stuffs, like baked goods, butter icing and custard. All of and at least a substantial portion of fat can be replaced by these mixtures. The amount which can be replaced depends on the application. The texture of the food stuff and the eating quality of the new product can thus be improved or remain unchanged.

A foodstuff composition comprising a mixture of water-soluble oligomers as described above may also comprise a fat ingredient, water and one or more additional ingredients selected from sweeteners, flours, emulsifiers, raising agents, thickeners, acidifiers and stabilizers.

More particularly a cake composition may also comprise:

(a) a fat ingredient;
(b) a sweetener;
(c) a flour;
(d) a raising agent;
(e) egg ingredient; and
(f) water.

An icing composition may also comprise:

(a) a fat ingredient;
(b) a sweetener; and
(c) water.

A mayonnaise composition may comprise:

(a) a fat ingredient;
(b) a thickener;
(c) an acidifier;
(e) an emulsifier; and
(f) water.

A spread composition may comprise:

(a) a fat ingredient;
(b) an emulsifier; and/or a stabilizer; and
(c) water.

Oligomeric mixtures and fractions thereof produced according to this invention can be used also as new low-caloric bulking agents. These mixtures can be used to replace carbohydrates such as sugar in different kinds of baked products or in other food stuffs. The amount of carbohydrate replaced with these mixtures depend on the application and average chain length of the oligomers.

By conventional means an initially degraded cellulose derivative mixture may be further separated into fraction of oligomers of differing average chain lengths. The viscosity of the various fractions will vary with the degree of average chain length of the oligomers contained within a fraction. Depending on the particular food stuff application, the invention further contemplates selecting one or more fractions from an initial oligomeric mixture having a viscosity (average chain length) which is most appropriate for the particular food stuff application. The selection of a particular average chain length fraction and the amount of such a fraction to be used in any given food stuff application may vary according to the amount of fat or carbohydrate to be replaced, it being recognized that the higher the absolute amount of substitution agent desired to be used in a particular foodstuff, the lower the viscosity (average molecular weight) of the fraction of mixture of oligomers should be used.

It is to be further recognized that an oligomeric mixture falling within a particular range of viscosites may be preferred in any particular food recipe insofar as it may be desirable to obtain an end product which resembles the eating quality of the normal recipe containing the normal relatively high level of fat or other high calorie ingredients.

The following examples 1-3 set forth typical exemplary routines for preparing a cellulase and various cellulose derivative hydrolysates therefrom.

Example 1 -- Typical Cellulase Preparation

The beta-glucosidase activity was removed by ion exchange chromatography from the commercially available cellulose preparation, Econase CE, as so designated by Alko Ltd., Helsinki, Finland which was produced from a strain of Trichoderma reesei. The cellulase preparation (column A, Table 1) was passed through a cation exchange column (S-Sepharose FF, Pharmacia, LKB Biotechnology AB, Uppsala, Sweden) which was equilibriated with 50mM sodium acetate pH 3.8 equilibrium buffer. The unbound protein (including oligomer producing endoglucanses) was washed out with the equilibrium buffer (column B, Table 1). Beta-glucosidase activity remained bound to the column and could be separately eluted with 1M NaCl.

TABLE 1

| Enzyme | Relative Enzyme Activity (%) | |
| --- | --- | --- |
| | A | B |
| | before ion exchange procedure | after ion exchange procedure |
| Beta-glucosidase | 100 | 0 |
| endo-1, 4, -beta-glucanase | 100 | 70 |

Endo- 1, 4- beta-glucanase and beta-glucosidase activities were measured as described by Bailey & Nevalainen (1981): Enzyme Microb. Technol. 3: 153-157. The relative enzyme activities reported in Table 1 of the Econase preparations before and after passage through an ion exchange column demonstrate the results of a typical means according to the invention of preparing an essentially beta-glucosidase free preparation for use in producing the oligomeric hydrolysates contemplated by the invention.

Although Table 1 reports relative enzyme activities, the absolute amount of enzyme used in any particular example is hereafter reported in terms of the amount of enzyme activity of the enzyme employed according to the universal activity unit of nano-katal (nkat) which stands for that amount of enzyme which produces one nanomole of reaction product in one second. (In the context of this application a hydrolysate reaction product such as an oligomer which is capable of reducing an agent such as dinitrosalicyclic acid which is reduced by the hydrolysate reaction product and subsequently measured.) The method of Bailey et al., Enzyme Microb, Technol., Vol. 9, pp. 153-157 describes how such measurements of enzyme activity can be made using glucose as a standard.

Example 2 -- Cellulose Derivative Enzymatic Hydrolyses

a. Methylcellulose hydrolysate

30g of methylcellulose (MC, Methocel MC, 64630, Fluka Chemie AG, CH-9470 Buchs, Switzerland) was mixed in 3l of water and the pH of the solution was adjusted to 5.5 with 15% phosphoric acid and the temperature was raised to 40°C. 0.3ml of the enzyme preparation having a total endo-1, 4 beta-glucanase activity of 1680 nkat from which the beta-glucosidase activity was removed chromatographically (as described in Example 1) was added to the solution. After hydrolysis for 24 hours the enzyme was inactivated by heating (90°C, 15 min.). The hydrolysate was subsequently cooled and freeze-dried.

The hydrolysate product contained less than 0.5% by weight of glucose and cellobiose.

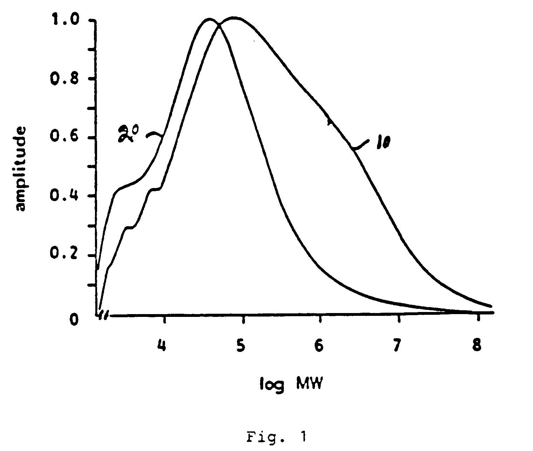

The molecular weight distribution patterns of methylcellulose, curve 10, and its hydrolysate, curve 20, are shown in FIG. 1.

The molecular weight distributions of the cellulose derivatives and their hydrolysates were determined by HPLC using a gel filtration column (TSK gel G2500PW, Toyo Soda Manufacturing Co., Ltd., Japan) with a refractive index detector (HP 1037 A) and Pharmacosmos Dextran Standards (Pharmacosmos, DK-4130, Viby Sj., Denmark). The eluent was 0.5M sodium chloride.

b. Hydroxypropylmethylcellulose Hydrolysate

20g of hydroxypropylmethylcellulose (HPMC, H-9262, Sigma Chemical Company, St. Louis, MO, U.S.A.) was mixed in 1l of water and the pH of the solution was adjusted to 5.5 with 15% phosphoric acid and the temperature was raised to 40°C. 0.24ml of the enzyme preparation having a total endo-1, 4 beta-glucanase activity of 1340 nkat from which the beta-glucosidase activity was removed chromatographically (as described in Example 1) was added to the solution. After two hours another 20g of hydroxypropylmethylcellulose was added to the solution. After the hydrolysis of 22 hours the enzyme was inactivated by heating (90°C, 15 min.). Finally the hydrolysate solution was cooled and freeze-dried.

The product contained less than 0.05% by weight of glucose and cellobiose.

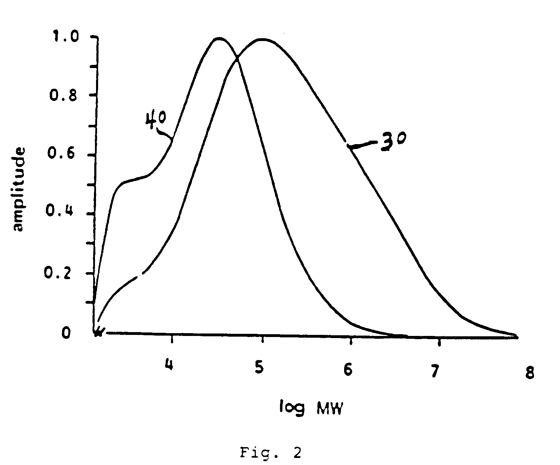

The molecular weight distribution patterns of the hydroxypropylmethylcellulose, curve 30, and its hydrolysate, curve 40, are shown in FIG. 2. The molecular weight distribution pattern was determined as described in Example 2A.

c. Carboxymethylcellulose Hydrolysate

## (i)    Hydrolysis with Trichoderma reesei derived enzyme preparation

20kg of carboxymethylcellulose (CMC 7MFD-type, a cellulose gum, also designated by the tradename Blanose and available from Hercules Chemical Company, 92507, Rueil-Malmaison Cedar, France; 7MFD designating a medium viscosity, food grade sodium carboxymethylcellulose having 7 out of 10 glucose units substituted with carboxymethyl) was mixed in 320l of water and the pH of the solution was adjusted to 5.5 with 15% phosphoric acid and the temperature was raised to 40°C. 0.27l of the enzyme preparation having a total endo-1, 4 beta-glucanase activity of 1,780,000 nkat from which the beta-glucosidase activity was removed chromatographically (as described in Example 1) was added to the CMC solution. After one hour another 20kg of CMC was added to the solution. After hydrolysis of 23 hours the enzyme was inactivated by heating (90°C, 15 min.). Finally, the hydrolysis solution was concentrated by conventional evaporating and spray-drying.

The product contained less than 2% by weight of glucose and cellobiose. When the same hydrolysis was carried out with the original cellulase enzyme preparation of Trichoderma reesei-fungus, the amount of produced glucose and cellobiose was above 5% by weight.

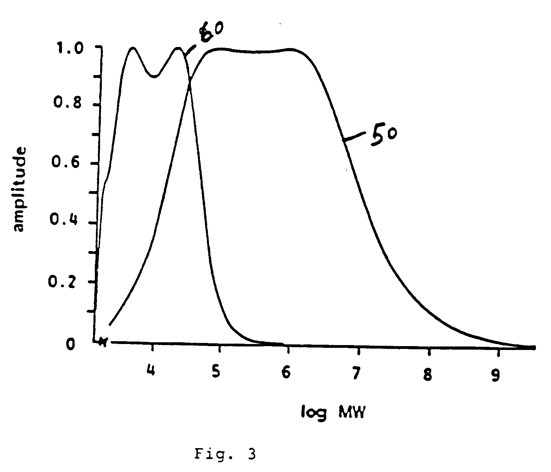

The molecular weight distribution patterns of carboxymethylcellulose, curve 50, and its hydrolysate, curve 60, are shown in FIG. 3.

The molecular weight distribution pattern was determined as described in Example 2a.

## (ii) Hydrolysis with Aspergillus and Penicillium derived enzyme preparations

The enzyme preparations selected were commercially available Cellulase AP 3 (Amano Pharmaceutical Co., Ltd., Nagoya, Japan) produced using an Aspergillus strain and Cellulase CP (Sturge Enzymes, North Yorkshire, England) produced using a Penicillium strain. Carboxymethylcellulose hydrolysates were prepared as described in Example 2c(i), except that 30g of CMC-7MFD was used in 1l of water, and the amounts of enzymes added were 0.028g of Cellulase AP 3 (having a total endo-1, 4 beta-glucanase activity of 1350 nkat) and 0.048g of Cellulase CP (having a total endo-1, 4 beta-glucanase activity of 1350 nkat). The viscosities and molecular weight distributions of the hydrolysates produced by either cellulase were similar (FIG. 3) to the hydrolysate produced with enzymes derived from Trichoderma reesei.

The viscosities of the various cellulose derivatives and their hydrolysates as described and prepared in Example 2 were measured using a Haake-Rotovisco viscometer with sensor systems NV (Karlsruhe, Federal Republic of Germany) (Table 2). The viscosities were measured in water solutions at 25°C. Table 2 sets forth the concentrations (by weight) of a variety of solutions all having the same viscosity.

TABLE 2

| Concentrations of cellulose derivatives and their respective hydrolysates in solution all having a viscosity of 20mPa.s (milli-Pascals-second) at 25°C. | |
| --- | --- |
| Cellulose Derivative | Concentration (by weight) |
| Methylcellulose | 2% |
| Methylcellulose hydrolysate | 5% |
| Hydroxypropylmethylcellulose | 3% |
| Hydroxypropylmethylcellulose hydrolysate | 10% |
| Carboxymethylcellulose | 2% |
| Carboxymethylcellulose hydrolysate | 20% |

As the data in Table 2 indicates, the hydrolysate of a cellulose derivative has a substantially lower viscosity than an equal amount by weight in aqueous solution of the cellulose derivative itself. Thus, the hydrolysate can be incorporated into a foodstuff in substantially higher quantity as a fat or sugar substitute than the cellulose derivative itself without compromising the texture, volume, density or the like of the foodstuff.

Example 3 The Fractionation of Carboxymethylcellulose Hydrolysate

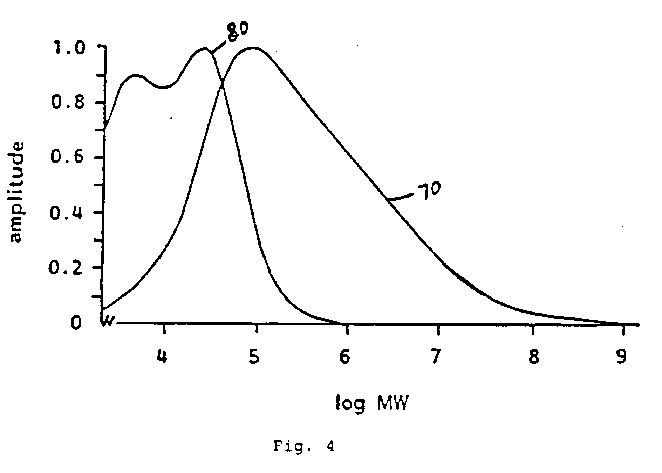

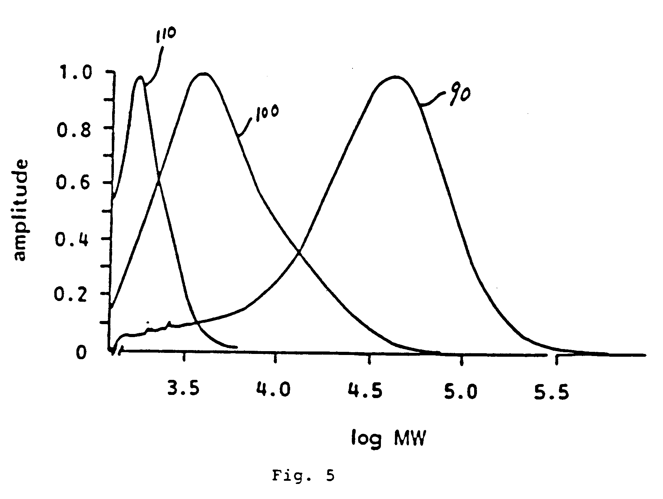

The carboxymethylcellulose hydrolysate was prepared as described in Example 2c(i), except that the raw material was CMC 7LFD (designating a low viscosity, food grade cellulose gum having 7 out of 10 glucose units substituted with carboxymethyl, designated under the tradename Blanose and available from Hercules Chemical Co., France) 1.6kg CMC was used in 8l of water and that the amount of enzyme added was 13.2ml having a total endo-1, 4 beta-glucanase activity of 87,000 nkat. 5ml of the hydrolysate (0.5g of dry matter) was further fractionated into three fractions by gel permeation chromatography (Pharmacia K 26/100 -column, Sephacryl S-200 -gel, Pharmacia Biotechnology AB, S-75182 Uppsala, Sweden). The eluent was distilled water, the flow rate was 14 ml/hour, and the fractionation process was carried out for 45 hours and fractions collected at intervals of 0.5 hours and pooled into three fractions (18 hours - 26 hours, curve 90, 26 hours - 32 hours, curve 100, and 32 hours - 38 hours, curve 110, FIG. 5, respectively). The molecular weight distributions of carboxymethylcellulose, curve 70, carboxymethylcellulose hydrolysate, curve 80, and the three further fractions, curves 90, 100, 110, FIGS. 4, 5, were determined by HPLC as described in Example 2.

Specific Exemplary Formulations wherein High Caloric Ingredient is Reduced

As described below a variety of popular high calorie food recipes were modified by substituting certain amounts of various carboxymethylcellulose hydrolysates for a certain portion of the normal level of a high calorie component of the food recipes. With respect to the invention, it is to be understood that the replacement of a high caloric ingredient is not limited to any particular cake, spread, icing, mayonnaise or other food recipe as may be specifically set forth herein for purposes of example. The various carboxymethylcellulose hydrolysates employed as substitutes or additives in the food recipes described hereinafter are referred to as EP151, EP151-2, EP151-49, EP151-51 and EP151-52 and the methods for preparing same were as follows:

a. CMC Hydrolysate EP151 was prepared as described in Example 2c(i) hereinabove.

b. CMC Hydrolysate EP151-2

20kg of carboxymethylcellulose (CMC 7LFD-type, a cellulose gum, also designated by the tradename Blanose and available from Hercules Chemical Company, 92507, Rueil-Malmaison Ceder, France, 7LFD designating a low viscosity, food grade sodium carboxymethylcellulose having 7 out of 10 glucose units substituted with carboxymethyl group) was mixed in 250l of water and the pH of the solution was adjusted to 5.8 with 15% phosphoric acid and the temperature raised to 40ºC. 0.177l of the above-described Trichoderma enzyme preparation, having a total endo-1, 4 beta-glucanase activity of 1,780,000 nkat, was added to the CMC solution. After one hour another 20kg of CMC was added to the solution. After hydrolysis for 23 hours the enzyme was inactivated by heating (90ºC, 15 min.). Finally, the hydrolysis solution was concentrated by spray-drying.

c. CMC Hydrolysate EP151-49

6kg of sodium carboxymethylcellulose (CMC Finnfix 5, available from Metsä-Serla, Chemical Division, SF-44100 Äänekoski, Finland, representing food grade purity and having a degree of substitution between 0.6-0.8) was mixed with 240l of water. The PH of the solution was adjusted between 5.5 and 5.9 with 15% of phosphoric acid and the temperature was maintained at 40ºC. 65ml of the above-described Trichoderma enzyme preparation, the endo-$\beta$-1,4-glucanase activity of which totalled 539,000 nkat, was added to the CMC solution. After an hour another 6kg of CMC was added. After hydrolysis for 23 hours, the enzyme was inactivated by heating the solution (90ºC, 15 min.). The hydrolysate was then concentrated by spray-drying.

d. CMC Hydrolysate EP151-51

6kg of sodium carboxymethylcellulose (CMC Finnfix 5) was mixed with 240l of water. Temperature and pH were as described with reference to preparation of EP151-49 (40°C, pH 5.5-5.9). 130ml of the Trichoderma enzyme preparation, the endo-$\beta$-1,4-glucanase activity of which totalled 1,079,000, was added to the CMC solution. After two hours another 6kg of CMC was added. After hydrolysis for 47 hours the enzyme was inactivated by heating the solution (90°C, 15 min.). The hydrolysate was then concentrated by evaporating and spray-drying.

e. CMC Hydrolysate EP151-52

This hydrolysate was produced as described with reference to EP151-51, except that 195 ml of the enzyme preparation containing an endo-$\beta$-1,4-glucanase activity of 1,618,000 nkat was used, and the hydrolysis time was 24 hours.

The viscosities, the intrinsic viscosities, the viscosity average molecular weights and the average degrees of polymerization of these various hydrolysate products are set forth in the following Table 3. The viscosities were determined using a rotational viscometer (Haake Viscotester VT 500 with sensor system NV, Karlsruhe, Federal Republic of Germany) . The intrinsic viscosities were measured according to the conventional method (described in Flory, Principles of Polymer Chemistry, Cornell Univ. Press, VII-4a, Ithaca, NY (1953)) at 25°C by using a calibrated Cannon Fenske capillary viscometer (size 50, Cannon Instrument, State College, PA, USA).

The viscosity average molecular weights of the CMC hydrolysates were calculated using the Mark-Houwink equation:

$$[\eta] = KM_v^a$$

where $[\eta]$ is intrinsic viscosity, $M_v$ is the average molecular weight of the polymer and K and a are hydrodynamic constants characteristic of the particular polymer-solvent system. The values of K and a for CMC, which were used in this study, were: K = 0.043 in 0.2M NaCl and a = 0.76 in 0.2M NaCl as described in Brown and Henley, Studies on Cellulose Derivatives Part IV. The Configuration of the Polyelectrolyte Sodium Chloride Solutions, Macromol. Chem., Vol. 79, pp. 68-88 (1964).

TABLE 3

| CMC Hydrolysate | Viscosity [1] (mPas) | Intrinsic Viscosity[2] (ml/g) | Average $M_v$ | Average DP |
|---|---|---|---|---|
| 151 | 32 | 31.4 | 7400 | 39 |
| 151-2 | 20 | 22.9 | 4800 | 25 |
| 151-49 | 23 | 18.4 | 3600 | 19 |
| 151-51 | 18 | 14.0 | 2500 | 13 |
| 151-52 | 18 | 14.3 | 2600 | 13 |

1) 20% (w.w) solution, 25°C shear rate = 584s$^{-1}$
2) measured in 0.2M NaCl, 25°C

It is noted that a variety of methods for determining average molecular weight exist, and therefore the values of average molecular weights determined, as well as the average DP values calculated from them, depend upon the experimental method and the basis of calculation. For example, the number average molecular weight can be determined by end group analysis, osmotic pressure, vapour pressure lowering, boiling point elevation and freezing point depression. The weight average molecular weight can be determined by light scattering experiment, the viscosity average molecular weight from the size exclusion chromatograph. All these methods can be used for determining the average DP values, although different results will be obtained depending on method and calculation used.

For purposes of the present invention, the average DP values were calculated from the viscosity average molecular weights, which were determined as described above (using K = 0.043 and a = 0.76 for calculation).

Madeira Cake

Following is a conventional ingredient recipe (representing a total batch weight) and method for making a Madeira cake having normal levels of sugar, fat and/or carbohydrate:

| Ingredients | Weight (g) |
|---|---|
| High ratio cake flour | 200 |
| Sugar - caster (sweetener) | 250 |
| High ratio shortening | 130 |
| Skimmed milk powder | 16 |
| Salt | 3 |
| Baking powder (raising agent) | 12 |
| Water | 180 |
| Egg | 176 |

METHOD (using Hobart Laboratory Mixer)

1. Place water, dry ingredients and fat in the bowl
2. Using beater, mix on speed 1 for 30 secs and scrape down
3. Mix on speed 3 for another 30 secs and scrape down
4. Add egg over 30 secs on speed 1 and scrape down
5. Mix on speed 2 until 0.8 specific gravity is obtained
6. Scale 180g into greased tins and bake at 170°C, middle shelf of domestic fan oven for 30 mins.

Utilizing various aqueous solutions of EP151, EP151-2, EP151-49, EP151-50 and EP151-51 of varying concentrations (and therefore, varying viscosity), 40% of the normal high ratio shortening (or fat) ingredient, i.e. 52g of fat or 5.4% of the total batch weight of the above Madeira cake recipe, was replaced with the following listed solutions in an amount so as to achieve a level of dry EP151, EP151-2, EP151-49, EP151-51 or EP151-52 ingredient (solids) as listed below (expressed as level of dry ingredient used as a percentage of total batch weight):

| CMC Hydrolysate Substitute (% by weight in aqueous solution) | Level of fat substitution (% of fat wt) | Level of fat substitution (% of total batch wt) | Level of dry ingredient used (% of total batch wt) |
|---|---|---|---|
| EP151 (40% solids) | 40% | 5.4% | 2.2% |
| EP151-2 (47% solids) | 40% | 5.4% | 2.5% |
| EP151-2 (40% solids) | 40% | 5.4% | 2.2% |
| EP151-49 (46% solids) | 40% | 5.4% | 2.5% |
| EP151-49 (40% solids) | 40% | 5.4% | 2.2% |
| EP151-51 (50% solids) | 40% | 5.4% | 2.7% |
| EP151-51 (40% solids) | 40% | 5.4% | 2.2% |
| EP151-51 (50% solids) | 40% | 5.4% | 2.7% |
| EP151-52 (40% solids) | 40% | 5.4% | 2.2% |

All of the Madeira cakes produced according to the above-listed substitutions had acceptable appearances, colors, volumes, textures, structures, odors, flavors and mouthfeels. The water activities of the various cakes differed slightly due to the slightly varying amounts and concentrations of the solutions substituted. Preparations wherein about 40% to about 75% of the fat ingredient is replaced with a substantially equivalent weight amount of a 40% solution of EP 151-51, should produce relatively acceptable cakes. Thus, it is believed that acceptable cakes may be obtained somewhere at a level of 40% - 75% fat replacement with a degraded cellulose derivative mixture of oligomers (average DP 3-100).

As is known in the art with regard to cake mixtures of the sort similar to the specific Madeira cake formulation set forth above, the amount of the major ingredients comprising more than about 10% of the total batch weight, may be varied by about plus or minus 5%, and the ingredients comprising less than about 10% of the total batch weight might typically be varied by about plus or minus 1%. With regard to cake mixtures generally (i.e. other than Madeira cake), flour, sugar (sweetener), shortening (fat), baking powder, water and egg components are typically common to all.

Butter Icing (Frosting)

Following is a conventional ingredient recipe (representing a total batch weight) and method for making a butter icing having normal levels of fat:

| Ingredients | Weight (g) |
|---|---|
| Butter (unsalted) | 179 |
| Icing sugar (sweetener) | 225 |
| Water | 96 |

METHOD

1. Soften the butter
1. Add icing sugar and cream together
3. Add water slowly and whisk until light and fluffy.

Again utilizing various solutions of EP151, EP151-2, EP151-49, EP151-51 and EP151-52 of varying concentrations, 33% of the fat (butter) ingredient, i.e. 60g or 11.8% of the total batch weight, was replaced with the following listed solutions wherein the following listed amounts of solid EP151, EP151-2, EP151-49, EP151-51 and EP151-52 were added (expressed as level of dry ingredient used as % of total batch weight):

| CMC Hydrolysate Substitute (% by weight of aqueous solution) | Level of fat substitution (% of fat wt) | Level of fat substitution (% of total batch wt) | Level of dry ingredient used (% of total batch wt) |
|---|---|---|---|
| EP151 (40% solids) | 33% | 11.8% | 4.7% |
| EP151-2 (47% solids) | 33% | 11.8% | 5.5% |
| EP151-2 (40% solids) | 33% | 11.8% | 4.7% |
| EP151-49 (46% solids) | 33% | 11.8% | 4.7% |
| EP151-49 (40% solids) | 33% | 11.8% | 4.7% |
| EP151-51 (50% solids) | 33% | 11.8% | 5.9% |
| EP151-51 (40% solids) | 33% | 11.8% | 4.7% |
| EP151-51 (50% solids) | 33% | 11.8% | 5.9% |
| EP151-52 (40% solids) | 33% | 11.8% | 4.7% |

All of the butter icings obtained via the above-listed levels of fat replacement were acceptable. Although these icings had slightly higher bulk densities relative to an icing obtained from a conventional recipe, the eating quality was not substantially affected. At 40% fat replacement with EP151-52 a better product was obtained than might be obtained via replacement of the fat with conventional fat sparing agents such as potato maltodextrin. Preparations in which about 30% to about 75% of the fat ingredient of the conventional recipe is substituted with an equivalent weight amount of a 40% solution of EP151-52 should produce relatively acceptable icings. Thus, a butter icing in which about 30% to about 75% of the fat ingredient is replaced with a degraded cellulose derivative oligomeric mixture having an average DP of between about 3 and about 100 produces a low calorie icing of acceptable eating quality. Where greater that about 50% fat replacement is desired, appropriate amounts of a stabilizer and/or an emulsifier should also preferably be included in the recipe.

As is known in the art, the tolerance range for variation of the various components in the typical icing formulation set forth above is plus or minus about 5%.

Mayonnaise

With respect to attempting to obtain a fat substituted low calorie mayonnaise product, the following is an already low calorie (fat reduced) recipe which might be modified by replacing a portion of the water and starch components of the low calorie recipe with an equivalent amount (by weight) of a solution of a degraded cellulose derivative.

| Ingredients | Weight (g) |
|---|---|
| Water | 243.0 |
| Vegetable oil | 180.00 |
| Instant starch (thickener) | 27.0 |
| White vinegar (acidifier) | 84.0 |
| Egg Yolk (emulsifier) | 48.0 |
| Salt | 9.0 |
| Powdered glucose | 9.0 |

METHOD

1. Blend all the dry ingredients and add slowly to the water which is being whipped on a Hobart at medium speed.
2. Heat the solution to 60ºC.
3. Cool to 20ºC.
4. Add the egg yolk and mix well.
5. Add the chilled oil (10ºC) slowly while agitating on the Hobart on medium speed.
6. When most of the oil is added, add vinegar slowly while mixing.

By way of example, one modified reduced calorie recipe of acceptable eating quality was obtained by replacing 54g of the water and 27g of the starch ingredients of the above mayonnaise recipe with an equivalent amount by weight of a 40% solution of EP151. EP151 may be incorporated into the above reduced calorie recipe up to at least about 15% of the total batch weight. A normal fat mayonnaise recipe typically includes about 2.5 times as much fat (vegetable oil) as the above recipe. Thus, an acceptable low fat mayonnaise can be obtained by replacing from about 25% to about 75% of the fat components with water and an appropriate amount of a degraded cellulose derivative mixture of oligomers having an average DP of between about 3 and about 100.

As is known in the art, mayonnaise formulations generally include at least water, vegetable oil (fat), vinegar (acidifier) and egg yolk (emulsifier); and the major components, water and fat, have a tolerance level of variation of plus or minus about 5%, and the minor components have a tolerance variation level of plus or minus about 1%.

Spreads

With respect to attempting to obtain a fat-reduced spread of better quality, the water component of the following already low fat spread recipe (representing a total batch weight) was modified by substituting a selected amount of a 50% solution of EP151-52 therefor.

| Ingredients | Weight (g) |
|---|---|
| Soft margarine blend (fat) | 234.0 |
| Dimodan CP (Emulsifier) | 6.0 |
| Water | 350.4 |
| Salt | 3.6 |
| Sobalg FD120 (Stablilizer) | 6.0 |

METHOD

1. Melt the fat blend and dissolve Dimodan CP into it. Heat to 50°C.
2. Dissolve the salt and Sobalg FD120 into the aqueous phase and heat to 50°C.
3. Place the warm fat phase in a large plastic beaker and insert the large paddle of the motor stirrer.
4. Add the aqueous phase to the fat phase slowly and gradually while stirring at a medium speed. Enough stirring is required to get a good dispersion, but care must be taken to ensure no air is drawn into the mixture.
5. Put the freezing unit of the ice cream maker on for 10 minutes before adding the emulsion.
6. Freeze down for 15 minutes, transfer into plastic tubs and immediately place in a constant temperature 5°C room.

Various levels of EP151-52 solution addition were tested by replacing 12g (2% of total batch weight) 60g (10% of total batch weight), and 120g (20% of total batch weight) of the water component in the above-listed low fat recipe with an equivalent amount by weight of a 50% solution of EP151-52. Up to 20% of total batch weight replacement (i.e. up to 120g) produced a low fat spread of acceptable eating quality. At about 20% total batch weight replacement, the EP151-52 containing low fat spread was better than the recipe above.

Normal fat containing spread typically includes about 79-83% of fat and about 16-20% water (as opposed to the above-listed low fat spread recipe wherein the fat phase is about 25-40% and water is about 58-75% water). Thus an acceptable low fat spread can be obtained by replacing from about 38% to about 75% of the fat components (typically butter and/or vegetable oils) in a normal fat margarine spread with water and an appropriate amount of a degraded cellulose derivative mixture of oligomers having an average DP of between about 3 and about 100. According to the above-described modification and improvement of the already low fat recipe, a normal fat spread recipe can thus suitably be modified by replacing from 38%-75% of the fat component with an equivalent amount by weight of an aqueous solution containing from about 3% to about 70% degraded cellulose derivative, and more preferably, a solution containing from about 30% to about 50% of degraded cellulose derivative material.

As is known in the art, all low fat spread formulations generally include at least fat blend, water, emulsifier and stabilizer ingredients; and, these various major components typically have a tolerance variation level of plus or minus about 1%.

16

Marzipan

Following is a conventional recipe (representing total batch weight) and method for making marzipan:

| Ingredients | Weight (g) |
|---|---|
| Icing sugar (carbohydrate) | 21.8 g |
| Caster sugar (carbohydrate) | 21.8 g |
| Ground Almonds | 43.7 g |
| Vanilla Flavoring | 0.8 g |
| Egg | 10.9 g |
| Lemon Juice | 0.8 g |

The above mixture is formed into a ball, lightly kneaded, rolled out and cut into desired shapes.

Up to about 40% of the icing sugar component may be replaced with an appropriate amount of a degraded cellulose derivative having an average DP of 3-100 and a confection of acceptable eating quality obtained.

Apart from sweet and savoury food products such as cake, icing, cookies, spreads, creams, snack fillings and the like, the degraded cellulose derivatives of the invention should be suitable as high calorie component (fat, carbohydrate) substitutes in relatively high protein containing systems such as meat pate and other meat emulsions, it being recognized that for any given food stuff composition, the particular range of amounts of certain ingredients of the normal high calorie recipe which may be modified to allow a degraded cellulose derivative to be incorporated, is determined to provide an end food product which has an eating quality approaching that of the normal high calorie recipe.

**Claims**

1.  A foodstuff composition comprising a mixture of water-soluble oligomers derived from enzymatic degradation of a water-soluble cellulose derivative which is carboxymethylcellulose, methylcellulose, methylethylcellulose, hydroxypropylcellulose, hydroxypropylmethylcellulose or a mixture thereof, substituted for at least a portion of a high calorie ingredient of the foodstuff composition, the said mixture of oligomers having an average degree of polymerization in the range of 3 to 100 and the average molecular weight in the range of 500-33,000 determined on the basis of intrinsic viscosity as described in the preamble to Table 3.

2.  A foodstuff composition according to claim 1 wherein the mixture of oligomers has an average degree of polymerization in the range of 5 to 100.

3.  A foodstuff composition according to claim 1 or 2 wherein the high calorie ingredient is a fat and/or a carbohydrate.

4.  A foodstuff composition according to any one of claims 1 to 3 wherein less than about 75% of the high calorie ingredient is removed from the food composition.

5.  A foodstuff composition according to claim 4 wherein at least about 25% of the high calorie ingredient is removed from the food composition.

6.  A foodstuff composition according to claim 4 or 5 wherein the high calorie ingredient is a fat and less than about 50% of the fat is removed from the food composition.

7.  A foodstuff composition according to any one of claims 1 to 6 comprising a fat ingredient, water and one or more additional ingredients selected from sweeteners, flours, emulsifiers, raising agents, thickeners, acidifiers and stabilizers.

8.  A foodstuff composition according to any one of claims 1 to 6 in the form of a cake composition comprising:
    (a) a fat ingredient;
    (b) a sweetener;

(c) a flour;
(d) a raising agent;
(e) egg ingredient; and
(f) water.

9.  A foodstuff composition according to any one of claims 1 to 6 in the form of an icing composition comprising:
    (a) a fat ingredient;
    (b) a sweetener; and
    (c) water.

10. A foodstuff composition according to any one of claims 1 to 6 in the form of a mayonnaise composition comprising:
    (a) a fat ingredient;
    (b) a thickener;
    (c) an acidifier;
    (d) an emulsifier; and
    (e) water.

11. A foodstuff composition according to any one of claims 1 to 6 in the form of a spread composition comprising:
    (a) a fat ingredient;
    (b) an emulsifier; and/or a stabilizer; and
    (c) water.

12. A foodstuff composition according to any one of claims 1 to 11 wherein the oligomeric mixture is produced by hydrolysing the cellulose derivative with an enzymatic cellulase preparation produced from microorganism selected from Trichoderma, Aspergillus and Penicillium.

13. A foodstuff composition according to claim 12 wherein the cellulose derivative is hydrolysed with a Trichoderma reesei cellulase preparation.

14. A method for preparing a low calorie food product comprising replacing at least a portion of a high calorie ingredient of a high calorie foodstuff composition by a mixture of water-soluble oligomers derived from enzymatic degradation of a water-soluble cellulose derivative which is carboxymethylcellulose, methylcellulose, methylethylcellulose, hydroxypropylcellulose, hydroxypropylmethylcellulose or a mixture thereof, substituted for at least a portion of a high calorie ingredient of the foodstuff composition, the mixture of oligomers having an average degree of polymerization in the range of 3 to 100 and the average molecular weight in the range of 500-33,000 determined on the basis of intrinsic viscosity.

15. A method according to claim 14 wherein the high calorie ingredient is a fat and/or a carbohydrate.

16. A method according to claim 14 or 15 wherein between about 25% and about 75% of the high calorie ingredient is replaced.

**Patentansprüche**

1.  Nahrungsmittelzusammensetzung, umfassend eine Mischung aus wasserlöslichen Oligomeren, abgeleitet aus dem enzymatischen Abbau eines wasserlöslichen Cellulosederivats, das Carboxymethylcellulose, Methylcellulose, Methylethylcellulose, Hydroxypropylcellulose, Hydroxypropylmethylcellulose oder eine Mischung davon ist, die wenigstens einen Teil eines Hochkalorienbestandteils der Nahrungsmittelzusammensetzung ersetzt, wobei die Oligomerenmischung einen durchschnittlichen Polymerisationsgrad im Bereich von 3 bis 100 und ein durchschnittliches Molekulargewicht im Bereich von 500 bis 33.000, bestimmt auf Basis der Intrinsik-Viskosität, wie in der Präambel zu Tabelle 3 beschrieben, hat.

2.  Nahrungsmittelzusammensetzung gemäß Anspruch 1, worin die Oligomerenmischung einen durchschnittlichen Polymerisationsgrad im Bereich von 5 bis 100 hat.

3. Nahrungsmittelzusammensetzung gemäß Anspruch 1 oder 2, worin der Hochkalorienbestandteil ein Fett und/oder ein Kohlenhydrat ist.

4. Nahrungsmittelzusammensetzung gemäß einem der Ansprüche 1 bis 3, worin weniger als etwa 75 % der Hochkalorienbestandteile aus der Nahrungsmittelzusammensetzung entfernt sind.

5. Nahrungsmittelzusammensetzung gemäß Anspruch 4, worin wenigstens etwa 25 % der Hochkalorienbestandteile aus der Nahrungsmittelzusammensetzung entfernt sind.

6. Nahrungsmittelzusammensetzung gemäß Anspruch 4 oder 5, worin der Hochkalorienbestandteil ein Fett ist, und weniger als ungefähr 50 % des Fetts von der Nahrungsmittelzusammensetzung entfernt sind.

7. Nahrungsmittelzusammensetzung gemäß einem der Ansprüche 1 bis 6, umfassend einen Fettbestandteil, Wasser und ein oder mehrere zusätzliche Bestandteile, ausgewählt aus Süßungsmitteln, Mehlen, Emulgatoren, Treibmitteln, Verdickungsmitteln, Säuerungsmitteln und Stabilisatoren.

8. Nahrungsmittelzusammensetzung gemäß einem der Ansprüche 1 bis 6 in Form einer Kuchenzusammensetzung, umfassend:
   (a) einen Fettbestandteil;
   (b) ein Süßungsmittel;
   (c) ein Mehl;
   (d) ein Treibmittel;
   (e) einen Eierbestandteil und
   (f) Wasser.

9. Nahrungsmittelzusammensetzung gemäß einem der Ansprüche 1 bis 6 in Form einer Zuckergußzusammensetzung, umfassend:
   (a) einen Fettbestandteil;
   (b) ein Lösungsmittel und
   (c) Wasser.

10. Nahrungsmittelzusammensetzung gemäß einem der Ansprüche 1 bis 6 in Form einer Mayonnaisezusammensetzung, umfassend:
    (a) einen Fettbestandteil;
    (b) ein Verdickungsmittel;
    (c) ein Säuerungsmittel;
    (d) einen Emulgator und
    (e) Wasser.

11. Nahrungsmittelzusammensetzung gemäß einem der Ansprüche 1 bis 6 in Form einer Aufstrichzusammensetzung, umfassend:
    (a) einen Fettbestandteil;
    (b) einen Emulgator und/oder Stabilisator und
    (c) Wasser.

12. Nahrungsmittelzusammensetzung gemäß einem der Ansprüche 1 bis 11, worin die Oligomerenmischung hergestellt ist durch Hydrolysieren des Cellulosederivats mit einer enzymatischen Cellulasezubereitung, hergestellt aus Mikroorganismen, ausgewählt aus **Trichoderma**, **Aspergillus** und Penicillium.

13. Nahrungsmittelzusammensetzung gemäß Anspruch 12, worin das Cellulosederivat mit einer **Trichoderma reesei** -Cellulasezubereitung hydrolysiert ist.

14. Verfahren zur Herstellung eines Niedrigkalorien-Nahrungsmittels, umfassend das Ersetzen wenigstens eines Teils eines Hochkalorienbestandteils einer hochkalorienreichen Nahrungsmittelzusammensetzung, durch eine Mischung von wasserlöslichen Oligomeren, die erhalten werden durch enzymatischen Abbau eines wasserlöslichen Cellulosederivats, nämlich Carboxymethylcellulose, Methylcellulose, Methylethylcellulose, Hydroxypropylcellulose, Hydroxypropylmethylcellulose oder Mischungen davon, wobei wenigstens ein Teil der Hochkalorienbestandteile der Nahrungsmittelzusammensetzung dadurch ersetzt wird, wobei die Mischung der Oligomeren einen Durchschnittspolymerisationsgrad im Bereich von 3 bis 100 und ein Durchschnittsmolekulargewicht im Bereich von 500 bis 33.000, bestimmt auf Basis der Intrinsikviskosität hat.

15. Verfahren gemäß Anspruch 14, wobei der Hochkalorienbestandteil ein Fett und/oder ein Kohlenhydrat ist.

16. Verfahren gemäß Anspruch 14 oder 15, wobei zwischen etwa 25 % und etwa 75 % des Hochkalorienbestandteils ersetzt ist.

**Revendications**

1. Composition alimentaire comprenant un mélange d'oligomères solubles dans l'eau, provenant de la dégradation enzymatique d'un dérivé de la cellulose soluble dans l'eau qui est la carboxyméthylcellulose, la méthylcellulose, la méthyléthylcellulose, l'hydroxypropylcellulose, l'hydroxypropylméthylcellulose ou un de leurs mélanges, remplaçant au moins une partie d'un ingrédient riche en calories de la composition alimentaire, ledit mélange d'oligomères ayant un degré moyen de polymérisation dans la gamme de 3 à 100 et un poids moléculaire moyen dans la gamme de 500 à 33 000, déterminé sur la base de la viscosité intrinsèque, comme décrit dans le préambule du tableau 3.

2. Composition alimentaire selon la revendication 1, dans laquelle le mélange d'oligomères a un degré moyen de polymérisation dans la gamme de 5 à 100.

3. Composition alimentaire selon revendication 1 ou 2, dans laquelle l'ingrédient riche en calories est une graisse et/ou un glucide.

4. Composition alimentaire selon l'une quelconque des revendications 1 à 3, dans laquelle moins d'environ 75% de l'ingrédient riche en calories sont éliminés de la composition alimentaire.

5. Composition alimentaire selon la revendication 4, dans laquelle au moins environ 25% de l'ingrédient riche en calories sont éliminés de la composition alimentaire.

6. Composition alimentaire selon la revendication 4 ou 5, où l'ingrédient riche en calories est une graisse et moins d'environ 50 % de la graisse sont éliminés de la composition alimentaire.

7. Composition alimentaire selon l'une quelconque des revendications 1 à 6, comprenant un ingrédient gras, de l'eau et un ou plusieurs ingrédients additionnels choisis parmi les édulcorants, les farines, les émulsifiants, les agents levants, les épaississants, les acidifiants et les stabilisants.

8. Composition alimentaire selon l'une quelconque des revendications 1 à 6, sous forme d'une composition de gâteau comprenant :
   (a) un ingrédient gras ;
   (b) un édulcorant ;
   (c) une farine ;
   (d) un agent levant ;
   (e) un ovoproduit ; et
   (f) de l'eau.

9. Composition alimentaire selon l'une quelconque des revendications 1 à 6, sous forme d'une composition de glaçage comprenant :
   (a) un ingrédient gras ;
   (b) un édulcorant ; et
   (c) de l'eau.

10. Composition alimentaire selon l'une quelconque des revendications 1 à 6, sous forme d'une composition de mayonnaise comprenant :
   (a) un ingrédient gras ;

    (b) un épaississant ;

    (c) un acidifiant ;

    (d) un émulsifiant ; et

    (e) de l'eau.

**11.** Composition alimentaire selon l'une quelconque des revendications 1 à 6, sous forme d'une composition à tartiner comprenant :

    (a) un ingrédient gras ;

    (b) un émulsifiant ; et/ou un stabilisant ; et

    (c) de l'eau.

**12.** Composition alimentaire selon l'une quelconque des revendications 1 à 11, dans laquelle le mélange oligomère est produit par hydrolyse du dérivé de la cellulose avec une préparation enzymatique de cellulase produite à partir d'un microorganisme choisi parmi Trichoderma, Aspergillus et Penicillium.

**13.** Composition alimentaire selon la revendication 12, dans laquelle le dérivé de la cellulose est hydrolysé avec une préparation de cellulase de Trichoderma reesei.

**14.** Procédé pour préparer un produit alimentaire hypocalorique, comprenant l'étape consistant à remplacer au moins une portion d'un ingrédient riche en calories d'une composition alimentaire riche en calories, par un mélange d'oligomères solubles dans l'eau provenant de la dégradation enzymatique d'un dérivé de la cellulose soluble dans l'eau qui est la carboxyméthylcellulose, la méthylcellulose, la méthyléthylcellulose, l'hydroxypropylcellulose, l'hydroxypropylméthylcellulose ou un de leurs mélanges, remplaçant au moins une partie d'un ingrédient riche en calories de la composition alimentaire, le mélange d'oligomères ayant un degré moyen de polymérisation dans la gamme de 3 à 100 et un poids moléculaire moyen dans la gamme de 500 à 33 000, déterminé sur la base de la viscosité intrinsèque.

**15.** Procédé selon la revendication 14, dans lequel l'ingrédient riche en calories est une graisse et/ou un glucide.

**16.** Procédé selon la revendication 14 ou 15, dans lequel on remplace environ 25 % à environ 75 % de l'ingrédient riche en calories.

Fig. 1

Fig. 2

Fig. 3

Fig. 4

Fig. 5